# EUROPEAN PATENT APPLICATION

(11) **EP 4 541 816 A1**
(43) Date of publication of application: **23.04.2025**
(21) Application number: 23823195.5
(22) Date of filing: 14.06.2023
(51) Int. Cl.: C07K 16/28, C07K 16/30, C07K 16/32, A61K 39/395

(54) **ANTI-CLDN18.2 ANTIBODY, AND PHARMACEUTICAL COMPOSITION AND USE THEREOF**

(30) Priority: 15.06.2022 CN 202210676083; 30.09.2022 CN 202211211390
(71) Applicant: Akeso Biopharma, Inc., Zhongshan, Guangdong 528437 (CN)
(72) Inventor: LI, Baiyong, Zhongshan, Guangdong 528437 (CN); XIA, Yu, Zhongshan, Guangdong 528437 (CN); WANG, Zhongmin, Zhongshan, Guangdong 528437 (CN); ZHANG, Peng, Zhongshan, Guangdong 528437 (CN)
(74) Representative: Cabinet Nony
(86) International application number: PCT/CN2023/100225
(87) International publication number: WO 2023/241629

(57) **Abstract**

Provided are an anti-CLDN18.2 antibody, and a pharmaceutical composition and the use thereof. Specifically, provided is an anti-CLDN18.2 antibody or an antigen-binding fragment thereof. The anti-CLDN18.2 antibody comprises a heavy chain variable region and a light chain variable region, the heavy chain variable region comprises HCDR1 to HCDR3, and the light chain variable region comprises LCDR1 to LCDR3, wherein the amino acid sequence of HCDR1 is as shown in SEQ ID NO: 5, the amino acid sequence of HCDR2 is as shown in SEQ ID NO: 6, and the amino acid sequence of HCDR3 is as shown in SEQ ID NO: 7; and the amino acid sequence of LCDR1 is as shown in SEQ ID NO: 8, the amino acid sequence of LCDR2 is as shown in SEQ ID NO: 9, and the amino acid sequence of LCDR3 is as shown in SEQ ID NO: 10. The anti-CLDN18.2 antibody has a good biological activity and anti-tumor application prospects.

## Description

### TECHNICAL FIELD

The present invention belongs to the field of biomedicine and relates to an anti-CLDN18.2 antibody and a pharmaceutical composition and use thereof.

### BACKGROUND

Tumor, especially a malignant tumor, is a serious health-threatening disease in the world today, and it is the second leading cause of death among various diseases. In recent years, the incidence of the disease has been increasing remarkably. The malignant tumor is characterized by poor treatment response, high late metastasis rate, and poor prognosis. Although conventional treatment methods (such as radiotherapy, chemotherapy, and surgical treatment) adopted clinically at present alleviate the pain to a great extent and prolong the survival time, the methods have great limitations, and it is difficult to further improve their efficacy.

The CLDN18.2 protein is an integrin membrane protein present in the epithelial and endothelial tight junction. It consists of 261 amino acids and is one of the Claudins (CLDNs) family members, and its N- and C-termini are both located in cells. The entire protein is expressed on the cell membrane. CLDN18.2 has 4 transmembrane domains, 2 extracellular loops, and 1 intracytoplasmic loop and is involved in the formation of a tight junction structure between cells (Gunzel, D.; Yu, A. S. L. Claudins and the Modulation of Tight Junction Permeability [J]. Physiological Reviews. 2013, 93(2), 525-569).

Extracellular loop 2 of the CLDN18.2 protein is a helix-turn-helix structure and forms a tight junction with the extracellular loop of the CLDN18.2 protein of an adjacent cell through hydrophobic bonds between aromatic residues. The extracellular loops of the CLDN18.2 proteins of adjacent cells can maintain the tight junction between the epithelial cell and the endothelial cell through interactions, regulate intercellular osmotic pressure, maintain the polarity of the epithelial cell and the endothelial cell, and participate in cell proliferation, and can participate in a variety of signal transduction pathways through the carboxyl-termini rich in serine, threonine, and tyrosine (Cao Chenxin, Research progress of transmembrane CLDN18.2 in targeted cancer therapy [J]. International Journal of Biologicals, 2020(01): 35-36-37-38-39-40).

The expression of the CLDN18.2 protein is highly restricted in normal healthy tissues and is only found in differentiated epithelial cells of the gastric mucosa, which is beneficial to maintaining the barrier function of the gastric mucosa. However, the CLDN18.2 protein is frequently abnormally changed during the development and progression of malignant tumors. For example, when gastric epithelial tissues are subjected to malignant transformation, the disturbance of cell polarity will result in the exposure of epitopes of the CLDN18.2 protein on the cell surface. At the same time, the CLDN18.2 gene is also abnormally activated, and it is highly selectively and stably expressed in specific tumor tissues, such as gastric cancer, affects the cell-cell and cell-extracellular matrix adhesion, and affects the maintenance of cell barrier function and polarity, thereby causing changes of intercellular cytokine and ion permeability, damaging the barrier function, and causing proliferative transformation of the cells (Hashimoto Itaru, Oshima Takashi, Claudins and Gastric Cancer: An Overview.[J]. Cancers (Basel), 2022, 14: undefined).

Claudiximab (IMAB362, zolbetuximab) is a human murine chimeric monoclonal antibody targeting CLDN18.2 developed by Ganymed, Germany. Claudiximab can specifically recognize the first extracellular domain (ECD1) outside the cell membrane of the CLDN18.2 protein and binds to it with high affinity, but does not bind to any other Claudin family member. Claudiximab mediates antibody-dependent cell-mediated cytotoxicity (ADCC) and complement-dependent cytotoxicity (CDC) to eliminate tumor cells. (Singh P, Toom S, Huang Y. Anti-claudin 18.2 antibody as new targeted therapy for advanced gastric cancer[J]. Journal of Hematology & Oncology, 2017, 10(1)).

Currently, there is a need to develop new anti-CLDN18.2 antibody drugs.

### SUMMARY

Through intensive studies and creative efforts, the inventors have obtained an anti-CLDN18.2 antibody. The inventors have surprisingly found that the anti-CLDN18.2 antibody of the present invention (also referred to as antibody or antibody of the present invention for short) has excellent affinity and/or specificity, can bind to CLDN18.2-expressing tumor cells with high specificity, and has good anti-tumor prospects. The present invention is detailed below.

One aspect of the present invention relates to an anti-CLDN18.2 antibody or an antigen-binding fragment thereof, wherein the anti-CLDN18.2 antibody comprises a heavy chain variable region comprising HCDR1 to HCDR3 and a light chain variable region comprising LCDR1 to LCDR3, wherein:
an amino acid sequence of HCDR1 is set forth in SEQ ID NO: 5, an amino acid sequence of HCDR2 is set forth in SEQ ID NO: 6, and an amino acid sequence of HCDR3 is set forth in SEQ ID NO: 7, and
an amino acid sequence of LCDR1 is set forth in SEQ ID NO: 8, an amino acid sequence of LCDR2 is set forth in SEQ ID NO: 9, and an amino acid sequence of LCDR3 is set forth in SEQ ID NO: 10.

In some embodiments of the present invention, the anti-CLDN18.2 antibody or the antigen-binding fragment thereof is provided, wherein,
an amino acid sequence of the heavy chain variable region of the anti-CLDN18.2 antibody is selected from SEQ ID NO: 2, SEQ ID NO: 11, SEQ ID NO: 13, and SEQ ID NO: 15; and
an amino acid sequence of the light chain variable region of the anti-CLDN18.2 antibody is selected from SEQ ID NO: 4, SEQ ID NO: 17, SEQ ID NO: 19, and SEQ ID NO: 21.

In some embodiments of the present invention, the anti-CLDN18.2 antibody or the antigen-binding fragment thereof is provided, wherein,
the amino acid sequence of the heavy chain variable region is set forth in SEQ ID NO: 2, and the amino acid sequence of the light chain variable region is set forth in SEQ ID NO: 4;
the amino acid sequence of the heavy chain variable region is set forth in SEQ ID NO: 11, and the amino acid sequence of the light chain variable region is set forth in SEQ ID NO: 17;
the amino acid sequence of the heavy chain variable region is set forth in SEQ ID NO: 11, and the amino acid sequence of the light chain variable region is set forth in SEQ ID NO: 19;
the amino acid sequence of the heavy chain variable region is set forth in SEQ ID NO: 11, and the amino acid sequence of the light chain variable region is set forth in SEQ ID NO: 21;
the amino acid sequence of the heavy chain variable region is set forth in SEQ ID NO: 13, and the amino acid sequence of the light chain variable region is set forth in SEQ ID NO: 17;
the amino acid sequence of the heavy chain variable region is set forth in SEQ ID NO: 13, and the amino acid sequence of the light chain variable region is set forth in SEQ ID NO: 19;
the amino acid sequence of the heavy chain variable region is set forth in SEQ ID NO: 13, and the amino acid sequence of the light chain variable region is set forth in SEQ ID NO: 21;
the amino acid sequence of the heavy chain variable region is set forth in SEQ ID NO: 15, and the amino acid sequence of the light chain variable region is set forth in SEQ ID NO: 17;
the amino acid sequence of the heavy chain variable region is set forth in SEQ ID NO: 15, and the amino acid sequence of the light chain variable region is set forth in SEQ ID NO: 19;
   or
the amino acid sequence of the heavy chain variable region is set forth in SEQ ID NO: 15, and the amino acid sequence of the light chain variable region is set forth in SEQ ID NO: 21.

In some embodiments of the present invention, the anti-CLDN18.2 antibody or the antigen-binding fragment thereof is provided, wherein for the antibody, a heavy chain constant region is an Ig gamma-1 chain C region (e.g., having an amino acid sequence set forth in SEQ ID NO: 23) or an Ig gamma-4 chain C region (e.g., NCBI ACCESSION: P01861.1), and a light chain constant region is an Ig kappa chain C region (e.g., having an amino acid sequence set forth in SEQ ID NO: 24).

In some embodiments of the present invention, the anti-CLDN18.2 antibody or the antigen-binding fragment thereof is provided, wherein the anti-CLDN18.2 antibody or the antigen-binding fragment thereof is selected from Fab, Fab', F(ab')₂, Fd, Fv, dAb, a complementarity determining region fragment, a single chain fragment variable, a humanized antibody, or a chimeric antibody.

In some embodiments of the present invention, the anti-CLDN18.2 antibody or the antigen-binding fragment thereof is provided, wherein,
the antibody comprises a non-CDR region derived from a non-murine species, such as from a human antibody.

In some embodiments of the present invention, the anti-CLDN18.2 antibody or the antigen-binding fragment thereof is provided, wherein an EC₅₀ for binding of the anti-CLDN18.2 antibody to a cell expressing CLDN18.2 is less than or equal to 0.5 µg/mL, less than or equal to 0.4 µg/mL, less than or equal to 0.35 µg/mL, less than or equal to 0.3 µg/mL, or less than or equal to 0.25 µg/mL; preferably, the EC₅₀ is measured by FACS (flow cytometry). In one embodiment of the present invention, the cell expressing CLDN18.2 is a CHO-K1 cell expressing CLDN18.2. In one embodiment of the present invention, the cell expressing CLDN18.2 is a CHO-K1 cell overexpressing CLDN18.2.

In some embodiments of the present invention, the anti-CLDN18.2 antibody is an anti-CLDN18.2 monoclonal antibody.

The present invention also relates to an anti-CLDN18.2 antibody or an antigen-binding fragment thereof, wherein the anti-CLDN18.2 antibody is a monoclonal antibody produced by a hybridoma cell line LT020 deposited at China Center for Type Culture Collection (CCTCC) with a CCTCC designation of CCTCC NO. C2022124.

The present invention also relates a hybridoma cell line LT020 deposited at China Center for Type Culture Collection (CCTCC) with a CCTCC designation of CCTCC NO. C2022124.

Another aspect of the present invention relates to an isolated nucleic acid molecule encoding the anti-CLDN18.2 antibody or the antigen-binding fragment thereof according to any aspect of the present invention.

Yet another aspect of the present invention relates to a recombinant vector comprising the isolated nucleic acid molecule of the present invention.

Yet another aspect of the present invention relates to a host cell comprising the isolated nucleic acid molecule of the present invention or the recombinant vector of the present invention.

Yet another aspect of the present invention relates to an antibody-drug conjugate comprising an antibody or an antigen-binding fragment thereof and a small molecule drug, wherein the antibody or the antigen-binding fragment thereof is the anti-CLDN18.2 antibody or the antigen-binding fragment thereof according to any aspect of the present invention; preferably, the small molecule drug is a small molecule cytotoxic drug; more preferably, the small molecule drug is an anti-tumor chemotherapeutic drug.

The chemotherapeutic drug may be a conventional anti-tumor chemotherapeutic drug, such as an alkylating agent, an antimetabolite, an anti-tumor antibiotic, a plant-based anticancer agent, a hormone, and an immunological agent.

In one or more embodiments of the present invention, the antibody-drug conjugate is provided, wherein the antibody or the antigen-binding fragment thereof is linked to the small molecule drug via a linker; the linker may be one known to those skilled in the art; for example, the linker is a hydrazone bond, a disulfide bond, or a peptide bond.

In one or more embodiments of the present invention, the antibody-drug conjugate is provided, wherein a molar ratio of the antibody or the antigen-binding fragment thereof to the small molecule drug is 1:(2-4), e.g., 1:2, 1:3, or 1:4.

Yet another aspect of the present invention relates to a pharmaceutical composition comprising an effective amount of the anti-CLDN18.2 antibody or the antigen-binding fragment thereof according to any aspect of the present invention or the antibody-drug conjugate according to any aspect of the present invention, wherein optionally, the pharmaceutical composition further comprises one or more pharmaceutically acceptable auxiliary materials.

Yet another aspect of the present invention relates to use of the anti-CLDN18.2 antibody or the antigen-binding fragment thereof according to any aspect of the present invention in preparing a medicament for treating or preventing a tumor;
preferably, the tumor is a CLDN18.2-positive tumor;
preferably, the tumor is one or more selected from biliary tract cancer, bronchogenic carcinoma, lymphoma, ovarian cancer, esophageal cancer, melanoma, a hematological malignancy, glioblastoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer.

The anti-CLDN18.2 antibody or the antigen-binding fragment thereof according to any aspect of the present invention for use in treating or preventing a tumor is provided;
preferably, the tumor is a CLDN18.2-positive tumor;
preferably, the tumor is selected from one or more of biliary tract cancer, bronchogenic carcinoma, lymphoma, ovarian cancer, esophageal cancer, melanoma, a hematological malignancy, glioblastoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer.

Yet another aspect of the present invention relates to a method for treating or preventing a tumor, which comprises the step of administering to a subject in need thereof an effective amount of the anti-CLDN18.2 antibody or the antigen-binding fragment thereof according to any aspect of the present invention; preferably, the tumor is a CLDN18.2-positive tumor;
preferably, the tumor is selected from one or more of biliary tract cancer, bronchogenic carcinoma, lymphoma, ovarian cancer, esophageal cancer, melanoma, a hematological malignancy, glioblastoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer.

In some embodiments of the present invention, the method for treating or preventing a tumor is provided, wherein drug administration is performed before or after surgery and/or before or after radiotherapy.

In some embodiments of the present invention, the method for treating or preventing a tumor is provided, wherein
a dose for each administration of the anti-CLDN18.2 antibody or the antigen-binding fragment thereof is 0.1-100 mg per kg body weight, preferably 5-50 mg or 5-15 mg per kg body weight;
preferably, drug administration is performed once every 3 days, every 4 days, every 5 days, every 6 days, every 10 days, every 1 week, every 2 weeks, or every 3 weeks;
preferably, a route of administration is intravenous drip infusion or intravenous injection.

In the present invention, unless otherwise defined, the scientific and technical terms used herein have the meanings generally understood by those skilled in the art. In addition, the laboratory operations of cell culture, molecular genetics, nucleic acid chemistry, and immunology used herein are the routine procedures widely used in the corresponding fields. Meanwhile, in order to better understand the present invention, the definitions and explanations of the relevant terms are provided below.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified into κ and λ light chains. Heavy chains are classified into µ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of the classical complement system. The VH and VL regions can be further subdivided into hypervariable regions (called complementarity determining regions (CDRs)), between which conservative regions called framework regions (FRs) are distributed. Each VH and VL consists of 3 CDRs and 4 FRs arranged from amino terminus to carboxyl terminus in the following order: FR1, CDR1, FR2, CDR2, FR3, CDR3, and FR4. The variable regions (VH and VL) of each heavy chain/light chain pair form an antibody-binding site. The assignment of amino acids to the regions or domains is based on Bethesda M.d., Kabat Sequences of Proteins of Immunological Interest (National Institutes of Health, (1987 and 1991)), or Chothia & Lesk J. Mol. Biol., 1987; 196: 901-917; Chothia et al., Nature, 1989; 342: 878-883, or the definition of the IMGT numbering system, see the definition in Ehrenmann F, Kaas Q, Lefranc M P., IMGT/3Dstructure-DB and IMGT/DomainGapAlign: a database and a tool for immunoglobulins or antibodies, T cell receptors, MHC, IgSF and MhcSF[J]., Nucleic acids research, 2009; 38(suppl_1): D301-D307.

The term "antibody" is not limited by any specific method for producing the antibody. For example, the antibody includes a recombinant antibody, a monoclonal antibody, and a polyclonal antibody. The antibody may be antibodies of different isotypes, such as IgG (e.g., subtype IgG1, IgG2, IgG3, or IgG4), IgA1, IgA2, IgD, IgE, or IgM.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technology first reported by Kohler et al. (Köhler G, Milstein C. Continuous cultures of fused cells secreting antibody of predefined specificity [J]. Nature, 1975; 256(5517): 495), but can also be obtained using recombinant DNA technology (see, e.g., U.S. Patent 4,816,567).

As used herein, the term "humanized antibody" refers to an antibody or an antibody fragment obtained when all or a part of CDR regions of a human immunoglobulin (receptor antibody) is replaced by the CDR regions of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321: 522-525; Reichmann et al., Nature, 1988; 332: 323-329; Presta, Curr. Op. Struct. Biol., 1992; 2: 593-596; and Clark, Immunol. Today, 2000; 21: 397-402.

As used herein, the term "single chain fragment variable (ScFv)" refers to a molecule comprising an antibody heavy chain variable region (VH) and an antibody light chain variable region (VL) linked via a linker. The VL and VH domains are paired to form a monovalent molecule by a linker that enables them to produce a single polypeptide chain (see, e.g., Bird et al., Science, 1988; 242:423-426 and Huston et al., Proc. Natl. Acad. Sci. USA, 1988; 85:5879-5883). Such scFv molecules may have the following general structures: NH2-VL-linker fragment-VH-COOH or NH2-VH-linker fragment-VL-COOH. An appropriate linker in the prior art consists of GGGGS amino acid sequence repeats or a variant thereof. For example, a linker having the amino acid sequence (GGGGS)4 may be used, but variants thereof may also be used (Holliger et al., Proc. Natl. Acad. Sci. USA, 1993; 90: 6444-6448). Other linkers that can be used in the present invention are described in Alfthan et al., Protein Eng., 1995; 8: 725-731, Choi et al., Eur. J. Immunol., 2001; 31:94-106, Hu et al., Cancer Res., 1996; 56: 3055-3061, Kipriyanov et al., J. Mol. Biol., 1999; 293: 41-56 and Roovers et al., Cancer Immunology, Immunotherapy, 2001, 50(1): 51-59.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction, or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as λ phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as *E. coli* or *bacillus subtilis,* fungal cells such as yeast cells or aspergillus, insect cells such as S2 drosophila cells or Sf9, or animal cells such as fibroblasts, CHO cells, GS cells, COS cells, NSO cells, HeLa cells, BHK cells, HEK 293 cells, or human cells.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity (K_{D}) of less than about 10⁻⁵ M, e.g., less than about 10⁻⁶ M, 10⁻⁷ M, 10⁻⁸ M, 10⁻⁹ M, or 10⁻¹⁰ M or less.

As used herein, the term "K_{D}" refers to a dissociation equilibrium constant for a specific antibody-antigen interaction and is used to describe the binding affinity between the antibody and the antigen. A smaller dissociation equilibrium constant indicates a stronger antibody-antigen binding and a higher affinity between the antibody and the antigen. Generally, antibodies bind to antigens (e.g., CLDN18.2 protein) with a dissociation equilibrium constant (K_{D}) of less than about 10⁻⁵ M, such as less than about 10⁻⁶ M, 10⁻⁷ M, 10⁻⁸ M, 10⁻⁹ M, or 10⁻¹⁰ M or less. K_{D} can be determined using methods known to those skilled in the art, e.g., using a Fortebio molecular interaction instrument.

As used herein, the terms "monoclonal antibody" and "mAb" have the same meaning and are used interchangeably; the terms "polyclonal antibody" and "pAb" have the same meaning and are used interchangeably. Besides, as used herein, amino acids are generally represented by single-letter and three-letter abbreviations known in the art. For example, alanine can be represented by A or Ala.

As used herein, the term "pharmaceutically acceptable carrier and/or excipient" refers to a carrier and/or an excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania, Mack Publishing Company, 1995), including but not limited to: pH regulators, surfactants, adjuvants, and ionic strength enhancers. For example, the pH regulators include, but are not limited to, phosphate buffer; the surfactants include, but are not limited to, cationic, anionic or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the onset of the disease (e.g., a tumor); a therapeutically effective amount refers to an amount sufficient to cure or at least partially stop the disease and complications thereof in patients suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight, and gender, the route of administration, and other treatments given concurrently, etc.

As used herein, when referring to the amino acid sequence of the CLDN18.2 protein (NCBI GenBank: NP_001002026.1), it includes the full length of the CLDN18.2 protein, or the extracellular fragment CLDN18.2 ECD of CLDN18.2, or a fragment comprising CLDN18.2 ECD, and it also includes a fusion protein of the full length of the CLDN18.2 protein or a fusion protein of CLDN18.2 ECD, such as a fragment fused to an Fc protein fragment of mouse or human IgG (mFc or hFc). However, those skilled in the art will appreciate that in the amino acid sequence of the CLDN18.2 protein, mutations or variations (including, but not limited to, substitutions, deletions, and/or additions) can be naturally produced or artificially introduced without affecting biological functions thereof. Therefore, in the present invention, the term "CLDN18.2 protein" shall include all such sequences, including their natural or artificial variants. In addition, when describing a sequence fragment of the CLDN18.2 protein, it also includes the corresponding sequence fragments in their natural or artificial variants.

Beneficial effects of the present invention

The present invention achieves one or more of the following effects:
(1) The anti-CLDN18.2 antibodies of the present invention have excellent affinity and specificity for CLDN18.2;
(2) The anti-CLDN18.2 antibodies of the present invention have high ADCC activity, CDC activity, and/or ADCP activity;
(3) The anti-CLDN18.2 antibodies of the present invention are capable of specifically killing CLDN18.2-positive cells.

### BRIEF DESCRIPTION OF THE DRAWINGS

FIG. 1: determination of binding activity of anti-CLDN18.2 specific antibody 8C5.1 for antigen on the surface of CHO-K1-CLDN18.2 cell by ELISA.
FIG. 2: determination of binding activity of anti-CLDN18.2 specific antibodies 8C5.1H1L1, 8C5.1H1L2, 8C5.1H2L1, and 8C5.1H2L2 for antigen on the surface of CHO-K1-CLDN18.2 cell by ELISA.
FIG. 3: determination of binding activity of anti-CLDN18.2 specific antibodies 8C5.1H2L3, 8C5.1H3L2, and 8C5.1H3L3 for antigen on the surface of CHO-K1-CLDN18.2 cell by ELISA.
FIG. 4: FACS assay of binding activity of anti-CLDN18.2 antibodies 8C5.1 and IMAB362 for CHO-K1-CLDN18.2 cell.
FIG. 5: FACS assay of binding activity of anti-CLDN18.2 antibodies 8C5.1H1L1, 8C5.1H1L2, 8C5.1H2L1, 8C5.1H2L2, 8C5.1H2L3, 8C5.1H3L2, 8C5.1H3L3, and IMAB362 for CHO-K1-CLDN18.2 cell.
FIG. 6: assay on ADCC activity of anti-CLDN18.2 antibodies for CHO-K1-CLDN18.2 cell.
FIG. 7: assay on CDC activity of anti-CLDN18.2 antibodies for CHO-K1-CLDN18.2 cell.
FIG. 8: MBMM's phagocytosis of CHO-K1-CLDN18.2 cells mediated by 8C5.1H3L3 and IMAB362.

Hybridoma cell line LT020 was deposited at China Center for Type Culture Collection (CCTCC) on May 19, 2022 with a CCTCC designation of CCTCC NO. C2022124, the depository address being Wuhan University, Wuhan, China, postal code: 430072.

Some sequences involved in the present invention are as follows:
8C5.1VH 
GAAGTGCAGCTGGTTGAGAGCGGCGGAGGACTGGTGAAACCTGGAGGATCTCTGAAGCTGA

GCTGCGCTGCTTCCGGATTCACTTTTAGCAACTCCGCCGTGAGCTGGGTGAGACAAACACCT
 
GAGAAGAGGCTGGAGTGGGTGGCTACAATCACAAGCGGCGTGAGCTACACCTACTACCTGG
ACTCCGTGAGGGGCAGATTCACAATCAGCAGGGACAACGCCAAGAACACCCTGTACCTGCA
GATGAGCAGCCTGAGGAGCGAGGATACCGCTATGTACTACTGTACCAGGCAGTTCAAGGGC
AACGCCCTGGATTACTGGGGCCAAGGAACCTCTGTGACAGTGAGCTCC(SEQ ID NO: 1)
 
EVQLVESGGGLVKPGGSLKLSCAASGFTFSNSAVSWVRQTPEKRLEWVATITSGVSYTYYLDSV
RGRFTISRDNAKNTLYLQMSSLRSEDTAMYYCTRQFKGNALDYWGQGTSVTVSS (SEQ ID NO: 2)
8C5.1VL 
GACATTGTGATGACCCAGTCCCCTTCCTCCCTGACAGTGACAGCTGGAGAGAAGGTGACAAT
GTCCTGCAAGAGCAGCCAGAGCCTGCTGAACTCCGGAAATCAGAAGAACTACCTGACCTGGT
ACCAGCAGAAGCCCGGACAACCACCTAAGCTGCTGATCTACTGGGCCTCCACCAGGGAAAG
CGGAGTTCCTGATAGATTCACCGGCAGCGGCTCCGGAACAGATTTCACACTGACAATCAACA
ACTTCCAGGCTGAGGACCTGGCCGTGTACTACTGTCAGAACGACTACTTCTACCCTCTGACCT
TCGGCGCCGGAACAAAGCTGGAACTGAAG (SEQ ID NO: 3)
 
DIVMTQSPSSLTVTAGEKVTMSCKSSQSLLNSGNQKNYLTWYQQKPGQPPKLLIYWASTRESGV
PDRFTGSGSGTDFTLTINNFQAEDLAVYYCQNDYFYPLTFGAGTKLELK (SEQ ID NO: 4)
8C5.1 CDR
   HCDR1: GFTFSNSA (SEQ ID NO: 5)
   HCDR2: ITSGVSYT (SEQ ID NO: 6)
   HCDR3: TRQFKGNALDY (SEQ ID NO: 7)
   LCDR1: QSLLNSGNQKNY (SEQ ID NO: 8)
   LCDR2: WAS (SEQ ID NO: 9)
   LCDR3: QNDYFYPLT (SEQ ID NO: 10)
8C5.1 H1VH 
EVQLVESGGGLVKPGGSLRLSCAASGFTFSNSAVSWVRQAPGKRLEWVATITSGVSYTYYLDSV
RGRFTISRDNAKNSLYLQMNSLRAEDTAVYYCTRQFKGNALDYWGQGTSVTVSS (SEQ ID NO:
11)
 
GAGGTGCAGCTGGTGGAGAGCGGCGGCGGCCTGGTGAAGCCCGGCGGCAGCCTGAGGCTGT
CCTGCGCCGCCTCTGGCTTCACCTTTTCTAACAGCGCCGTGTCTTGGGTGAGGCAGGCACCTG
GCAAGCGCCTGGAGTGGGTGGCCACCATCACATCCGGCGTGTCTTACACCTACTATCTGGAC
AGCGTGCGGGGCAGATTCACAATCAGCAGAGATAACGCCAAGAACAGCCTGTATCTGCAGA
TGAACTCTCTGCGGGCCGAGGACACCGCCGTGTACTATTGTACAAGACAGTTTAAGGGCAAT
GCCCTGGATTACTGGGGCCAGGGCACCAGCGTGACAGTGAGCTCC (SEQ ID NO: 12)
8C5.1 H2VH 
EVQLVESGGGLVKPGGSLRLSCAASGFTFSNSAVSWVRQAPGKGLEWVSTITSGVSYTYYADSV
KGRFTISRDNAKNSLYLQMNSLRAEDTAVYYCTRQFKGNALDYWGQGTSVTVSS (SEQ ID NO:
13)
 
GAGGTGCAGCTGGTGGAGAGCGGCGGCGGCCTGGTGAAGCCCGGCGGCAGCCTGAGGCTGT
CCTGCGCAGCATCTGGCTTCACCTTTTCTAACAGCGCCGTGTCTTGGGTGAGGCAGGCACCTG
GCAAGGGACTGGAGTGGGTGAGCACCATCACATCCGGCGTGTCTTACACCTACTATGCCGAC
TCCGTGAAGGGCCGGTTCACAATCAGCAGAGATAACGCCAAGAACAGCCTGTATCTGCAGAT
GAACTCTCTGAGGGCCGAGGACACCGCCGTGTACTATTGTACACGCCAGTTTAAGGGCAATG
CCCTGGATTACTGGGGCCAGGGCACCAGCGTGACAGTGAGCTCC (SEQ ID NO: 14)
8C5.1 H3VH 
EVQLVESGGGLVKPGGSLRLSCAASGFTFSNSAVSWVRQAPGKGLEWVSSITSGVSYTYYADSV
KGRFTISRDNAKNSLYLQMNSLRAEDTAVYYCTRQFKGNALDYWGQGTSVTVSS (SEQ ID NO:
15)
 
GAGGTGCAGCTGGTGGAGAGCGGCGGCGGCCTGGTGAAGCCCGGCGGCAGCCTGAGGCTGT
CCTGCGCAGCATCTGGCTTCACCTTTTCTAACAGCGCCGTGTCTTGGGTGAGGCAGGCACCTG
GCAAGGGACTGGAGTGGGTGAGCTCCATCACATCCGGCGTGTCTTACACCTACTATGCCGAC
TCCGTGAAGGGCCGGTTCACAATCAGCAGAGATAACGCCAAGAACAGCCTGTATCTGCAGAT
GAACTCTCTGAGGGCCGAGGACACCGCCGTGTACTATTGTACACGCCAGTTTAAGGGCAATG
CCCTGGATTACTGGGGCCAGGGCACCAGCGTGACAGTGAGCTCC (SEQ ID NO: 16)
8C5.1 L1VL 
DIVMTQSPSSLSVSPGERVTMSCKSSQSLLNSGNQKNYLTWYQQKPGQPPKLLIYWASTRESGVP

DRFSGSGSGTDFTLTINSFQAEDVAVYYCQNDYFYPLTFGAGTKLELK (SEQ ID NO: 17)
 
GATATCGTGATGACCCAGAGCCCATCTAGCCTGTCCGTGTCTCCAGGCGAGAGAGTGACCAT
GAGCTGCAAGTCTTCCCAGAGCCTGCTGAATAGCGGCAACCAGAAGAACTACCTGACATGGT
ACCAGCAGAAGCCAGGCCAGCCTCCAAAGCTGCTGATCTACTGGGCCAGCACAAGAGAGAG
CGGCGTGCCAGACAGATTCTCTGGCAGCGGCTCTGGCACCGACTTCACACTGACCATCAACT
CCTTCCAGGCCGAGGATGTGGCCGTGTATTACTGCCAGAACGATTACTTCTATCCCCTGACAT
TCGGCGCCGGCACAAAGCTGGAGCTGAAG (SEQ ID NO: 18)
8C5.1 L2VL 
DIVMTQSPSSLSVSPGERVTISCKSSQSLLNSGNQKNYLTWYQQKPGQPPKLLIYWASTRESGVPD

RFSGSGSGTDFTLTISSFQAEDVAVYYCQNDYFYPLTFGAGTKLELK (SEQ ID NO: 19)
 
GACATCGTGATGACCCAGTCTCCAAGCTCCCTGAGCGTGTCCCCAGGAGAGAGGGTGACAAT
CTCCTGCAAGTCTAGCCAGTCTCTGCTGAACAGCGGCAATCAGAAGAACTACCTGACCTGGT
ATCAGCAGAAGCCTGGCCAGCCCCCTAAGCTGCTGATCTACTGGGCCTCCACCAGGGAGTCT
GGAGTGCCAGACAGATTTTCTGGCAGCGGCTCCGGCACAGATTTCACCCTGACAATCTCCTCT
TTTCAGGCCGAGGACGTGGCCGTGTACTATTGTCAGAATGATTACTTCTATCCCCTGACCTTT
GGCGCCGGCACAAAGCTGGAGCTGAAG (SEQ ID NO: 20)
8C5.1 L3VL 
DIVMTQSPSSLSVSPGERVTISCKSSQSLLNSGNQKNYLAWYQQKPGQPPKLLIYWASTRESGVPD
RFSGSGSGTDFTLTISSLQAEDVAVYYCQNDYFYPLTFGAGTKLELK (SEQ ID NO: 21)
 
GACATCGTGATGACCCAGTCTCCAAGCTCCCTGAGCGTGTCCCCAGGAGAGAGGGTGACAAT
CTCCTGCAAGTCTAGCCAGTCTCTGCTGAACAGCGGCAATCAGAAGAACTACCTGGCCTGGT
ATCAGCAGAAGCCTGGCCAGCCCCCTAAGCTGCTGATCTACTGGGCCTCCACCAGGGAGTCT
GGAGTGCCAGACAGATTCTCTGGCAGCGGCTCCGGCACAGACTTCACCCTGACAATCTCCTC
TCTGCAGGCCGAGGACGTGGCCGTGTACTATTGTCAGAATGATTACTTCTATCCCCTGACCTT
TGGCGCCGGCACAAAGCTGGAGCTGAAG (SEQ ID NO: 22)
Amino acid sequence of the heavy chain constant region of hG1WT 
ASTKGPSVFPLAPSSKSTSGGTAALGCLVKDYFPEPVTVSWNSGALTSGVHTFPAVLQSSGLYSLS
SVVTVPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSLTCLVKGFYPSDI
AVEWESNGQPENNYKTTPPVLDSDGSFFLYSKLTVDKSRWQQGNVFSCSVMHEALHNHYTQKS
LSLSPGK (SEQ ID NO: 23)
Amino acid sequence of light chain constant region 


 (SEQ ID NO: 24)
Amino acid sequence of heavy chain of positive control antibody IMAB362: 
QVQLQQPGAELVRPGASVKLSCKASGYTFTSYWINWVKQRPGQGLEWIGNIYPSDSYTNYNQKF
KDKATLTVDKSSSTAYMQLSSPTSEDSAVYYCTRSWRGNSFDYWGQGTTLTVSSASTKGPSVFP
LAPSSKSTSGGTAALGCLVKDYFPEPVTVSWNSGALTSGVHTFPAVLQSSGLYSLSSVVTVPSSSL
GTQTYICNVNHKPSNTKVDKRVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSLSPGK (SEQ
ID NO: 25)
Amino acid sequence of light chain of positive control antibody IMAB362:

DIVMTQSPSSLTVTAGEKVTMSCKSSQSLLNSGNQKNYLTWYQQKPGQPPKLLIYWASTRESGV
PDRFTGSGSGTDFTLTISSVQAEDLAVYYCQNDYSYPFTFGSGTKLEIKRTVAAPSVFIFPPSDEQL
KSGTASVVCLLNNFYPREAKVQWKVDNALQSGNSQESVTEQDSKDSTYSLSSTLTLSKADYEKH
KVYACEVTHQGLSSPVTKSFNRGEC (SEQ ID NO: 26)

### DETAILED DESCRIPTION

Embodiments of the present invention will be described in detail below with reference to examples, but those skilled in the art will appreciate that the following examples are only for illustrating the present invention, and should not be construed as limitations to the scope of the present invention. Experimental procedures without specified conditions in the examples are conducted according to conventional conditions or conditions recommended by the manufacturer. Reagents or instruments used are all commercially available conventional products if the manufacturers thereof are not specified.

The cell line CHO-K1-CLDN18.2 was constructed by Akeso Biopharma Inc. The cell line CHO-K1-CLDN18.2 was prepared by viral infection of CHO-K1 cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was pCDH-hCLDN18.2FL-Puro (CLDN18.2, Genebank ID: NP_001002026.1, vector pCDH-CMV-MCS-EF1-Puro, purchased from Youbio, Cat. No.: VT1480).

### Preparation Example 1: Preparation of Anti-CLDN18.2 Antibody 8C5.1

### 1. Preparation of hybridoma cell line LT020

The immunogen used for the preparation of anti-CLDN18.2 antibody was a 3T3 cell line overexpressing human Claudin18.2 (Genbank ID: NP-001002026.1) (Chinese Academy of Sciences) (3T3 hCLDN18.2). Spleen cells of immunized mice were fused with myeloma cells of mice to prepare hybridoma cells. With the CHO-K1 cell line overexpressing human Claudin18.2 ( Institute of Basic Medical Sciences, Chinese Academy of Medical Sciences) (CHO-K1-hCLDN18.2) used as an antigen, the hybridoma cells were screened by indirect ELISA to obtain a hybridoma cell capable of secreting the antibody specifically binding to CLDN18.2. The hybridoma cell obtained by screening was subjected to limiting dilution cloning to obtain a stable hybridoma cell line. The hybridoma cell line above was named hybridoma cell line LT020, and the monoclonal antibody secreted by the hybridoma cell line was named 8C5.1.

Hybridoma cell line LT020 was deposited at China Center for Type Culture Collection (CCTCC) on May 19, 2022 with a CCTCC designation of CCTCC NO. C2022124, the depository address being Wuhan University, Wuhan, China, postal code: 430072.

### 2. Preparation of Anti-CLDN18.2 Antibody 8C5.1

The cell line LT020 prepared above was cultured in a CD Medium (Chemical Defined Medium, containing 4% Glutamax (Gibco 35050079) and 1% penicillin-streptomycin (Gibco 15140163)) at 5% CO₂ and 37 °C. After 7 days, the cell culture supernatant was collected, subjected to high-speed centrifugation and vacuum filtration through a microfiltration membrane, and purified by using a HiTrap protein A HP column to obtain the antibody 8C5.1.

### Preparation Example 2: Sequence Analysis of Anti-CLDN18.2 Antibody 8C5.1

mRNA was extracted from the cell line LT020 cultured in Preparation Example 1 according to the method described in the manual of RNAprep pure Cell/Bacteria Kit (Tiangen, Cat. No.: DP430).

cDNA was synthesized according to the manual of Invitrogen SuperScript^{®} III First-Strand Synthesis System for RT-PCR kit and amplified by PCR.

The PCR-amplified product was directly subjected to TA cloning according to the manual of the pEASY-T1 Cloning Kit (Transgen CT101).

The TA cloning product was directly sequenced, and the sequencing results are as follows:
The nucleotide sequence of the heavy chain variable region is set forth in SEQ ID NO: 1 with a length of 354 bp.

The encoded amino acid sequence is set forth in SEQ ID NO: 2 with a length of 118 amino acids.

The nucleotide sequence of the light chain variable region is set forth in SEQ ID NO: 3 with a length of 339 bp.

The encoded amino acid sequence is set forth in SEQ ID NO: 4 with a length of 113 amino acids.

The 6 CDRs of the antibody 8C5.1 defined by the IMGT numbering system are as follows:
for the heavy chain, the sequence of HCDR1 is set forth in SEQ ID NO: 5, the sequence of HCDR2 is set forth in SEQ ID NO: 6, and the sequence of HCDR3 is set forth in SEQ ID NO: 7;
for the light chain, the sequence of LCDR1 is set forth in SEQ ID NO: 8, the sequence of LCDR2 is set forth in SEQ ID NO: 9, and the sequence of LCDR3 is set forth in SEQ ID NO: 10.

### Preparation Example 3: Light and Heavy Chain Design and Preparation of Humanized Anti-Human CLDN18.2 Antibodies

### 1. Light and heavy chain design of humanized anti-human CLDN18.2 antibodies 8C5.1H1L1, 8C5.1H1L2, 8C5.1H2L1, 8C5.1H2L2, 8C5.1H2L3, 8C5.1H3L2, and 8C5.1H3L3

Based on the sequences of the antibody 8C5 obtained in Preparation Example 2, the antibody model was simulated by a computer and mutations were designed according to the model to obtain the sequences of the variable regions of antibodies 8C5.1H1L1, 8C5.1H1L2, 8C5.1H2L1, 8C5.1H2L2, 8C5.1H2L3, 8C5.1H3L2, and 8C5.1H3L3 (for the antibodies, the heavy chain constant regions were all the Ig gamma-1 chain C region, SEQ ID NO: 23, and the light chain constant regions were all the Ig kappa chain C region, SEQ ID NO: 24).

The designed variable region sequences are shown in Table 1 below.

**Table 1**

| No. | Name of antibody | Amino acid sequence of heavy chain variable region SEQ ID NO: | Nucleotide sequence of heavy chain variable region SEQ ID NO: | Amino acid sequence of light chain variable region SEQ ID NO: | Nucleotide sequence of light chain variable region SEQ ID NO: |
|---|---|---|---|---|---|
| 1 | 8C5.1H1L1 | 11 | 12 | 17 | 18 |
| 2 | 8C5.1H1L2 | 11 | 12 | 19 | 20 |
| 3 | 8C5.1H2L1 | 13 | 14 | 17 | 18 |
| 4 | 8C5.1H2L2 | 13 | 14 | 19 | 20 |
| 5 | 8C5.1H2L3 | 13 | 14 | 21 | 22 |
| 6 | 8C5.1H3L2 | 15 | 16 | 19 | 20 |
| 7 | 8C5.1H3L3 | 15 | 16 | 21 | 22 |

For each of the above 7 antibodies 8C5.1H1L1, 8C5.1H1L2, 8C5.1H2L1, 8C5.1H2L2, 8C5.1H2L3, 8C5.1H3L2, and 8C5.1H3L3, the length of the nucleotide sequence of the heavy chain variable region was 354 bp and the length of the encoded amino acid sequence was 118 aa, and the length of the nucleotide sequence of the light chain variable region was 339 bp and the length of the encoded amino acid sequence was 113 aa.

Besides, the above 7 antibodies had the same HCDR1-HCDR3 and LCDR1-LCDR3 shown below:
the sequence of HCDR1 is set forth in SEQ ID NO: 5, the sequence of HCDR2 is set forth in SEQ ID NO: 6, and the sequence of HCDR3 is set forth in SEQ ID NO: 7;
the sequence of LCDR1 is set forth in SEQ ID NO: 8, the sequence of LCDR2 is set forth in SEQ ID NO: 9, and the sequence of LCDR3 is set forth in SEQ ID NO: 10.

### 2. Preparation of humanized antibodies 8C5.1H1L1, 8C5.1H1L2, 8C5.1H2L1, 8C5.1H2L2, 8C5.1H2L3, 8C5.1H3L2, and 8C5.1H3L3

The heavy chain constant regions were all the Ig gamma-1 chain C region, SEQ ID NO: 23; the light chain constant regions were all the Ig kappa chain C region, ACCESSION: P01834, SEQ ID NO: 24.

The heavy chain cDNA and light chain cDNA of each of 8C5.1H1L1, 8C5.1H1L2, 8C5.1H2L1, 8C5.1H2L2, 8C5.1H2L3, 8C5.1H3L2, and 8C5.1H3L3 were cloned into a pUC57simple (provided by GenScript) vector to respectively obtain: pUC57simple-8C5.1H1, pUC57simple-8C5.1L1;
pUC57simple-8C5.1H1, pUC57simple-8C5.1L2;
pUC57simple-8C5.1H2, pUC57simple-8C5.1L1;
pUC57simple-8C5.1H2, pUC57simple-8C5.1L2;
pUC57simple-8C5.1H2, pUC57simple-8C5.1L3;
pUC57simple-8C5.1H3, pUC57simple-8C5.1L2; and
pUC57simple-8C5.1H3, pUC57simple-8C5.1L3.

According to the standard techniques described in *Molecular Cloning: A Laboratory Manual (Second Edition),* EcoRI&HindIII was used to digest the synthesized full-length heavy and light chain genes. The genes were subcloned into the expression vector pcDNA3.1 by the digestion of the restriction enzyme (EcoRI&HindIII) to obtain expression plasmids pcDNA3.1-8C5.1H1, pcDNA3.1-8C5.1L1, pcDNA3.1-8C5.1H2, pcDNA3.1-8C5.1L2, pcDNA3.1-8C5.1H3, and pcDNA3.1-8C5.1L3, and further the heavy/light chain genes of the recombinant expression plasmids were sequenced. Then the designed gene combinations comprising the corresponding light and heavy chain recombinant plasmids (pcDNA3.1-8C5.1H1/pcDNA3.1-8C5.1L1,
pcDNA3.1-8C5.1H1/pcDNA3.1-8C5.1L2,
pcDNA3.1-8C5.1H2/pcDNA3.1-8C5.1L1,
pcDNA3.1-8C5.1H2/pcDNA3.1-8C5.1L2,
pcDNA3.1-8C5.1H2/pcDNA3.1-8C5.1L3,
pcDNA3.1-8C5.1H3/pcDNA3.1-8C5.1L2, and
pcDNA3.1-8C5.1H3/pcDNA3.1-8C5.1L3) were each co-transfected into 293F cells, and the culture solutions were collected and purified. After the sequences were verified to be correct by sequencing, endotoxin-free expression plasmids were prepared and transiently transfected into HEK293 cells for antibody expression. After 7 days, the cell culture solutions were collected and subjected to affinity purification on a Protein A column to obtain humanized antibodies.

### Example 1: Determination of Binding Activity of Anti-CLDN18.2 Specific Antibodies for Antigen by ELISA

CHO-K1-CLDN18.2 cells (Chinese hamster ovary cells CHO-K1 overexpressing CLDN18.2, CHO-K1 purchased from Cell Resource Center, Institute of Basic Medical Sciences, Chinese Academy of Medical Sciences, Cat. No.: 3111C0001CCC000004) were plated on a cell plate (Corning, 3599) at 2 × 10⁴ cells/well and cultured overnight at 5% CO₂ and 37 °C, and then the cell plate in which the cells were incubated was washed once with PBST and blocked for 2 h at 37 °C using PBST containing 1% BSA as a blocking solution. After blocking, the plate was washed 3 times with PBST. The antibodies serially diluted with PBST solution (the dilution gradients for the antibody are shown in Table 2) were added. The ELISA plate containing the test antibodies was incubated at 37 °C for 30 min and then washed 3 times with PBST. After washing, for the murine antibody, a working solution of the HRP-labeled goat anti-mouse FC (H+L) (Jackson, Cat. No.: 109-035-098) secondary antibody diluted at a ratio of 1:5000 was added, and for the humanized antibodies and IMAB362, a working solution of an HRP-labeled goat anti-human IgG (H+L) (Jackson, Cat. No.: 109-035-088) secondary antibody diluted at a ratio of 1:5000 was added. Then, the plate was incubated at 37 °C for 30 min. After incubation, the plate was washed 4 times with PBST, TMB (Neogen, 308177) was added for color developing in the dark for 5 min, and then a stop solution was added to terminate the chromogenic reaction. The ELISA plate was put into a microplate reader immediately, and the OD value of each well in the ELISA plate was read at 450 nm. The data were analyzed and processed by SoftMax Pro 6.2.1.

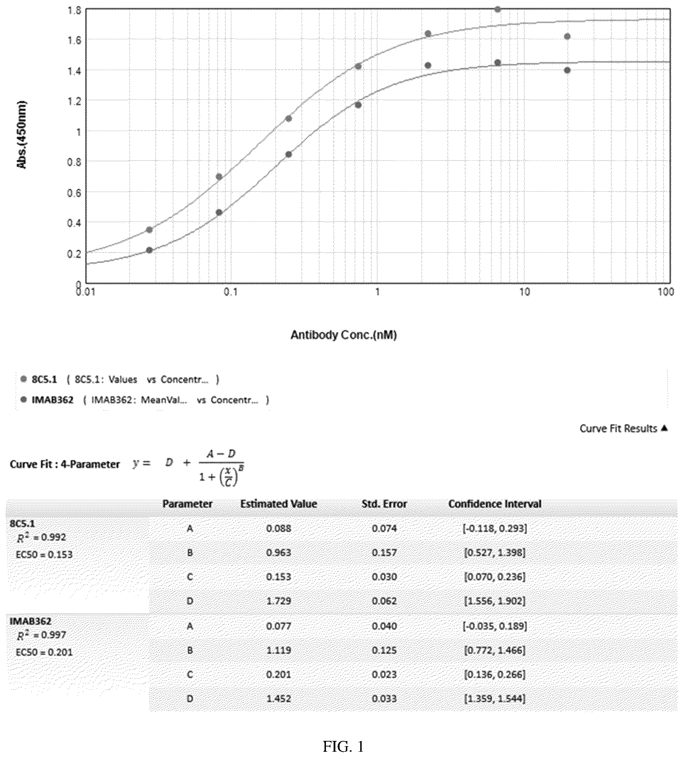

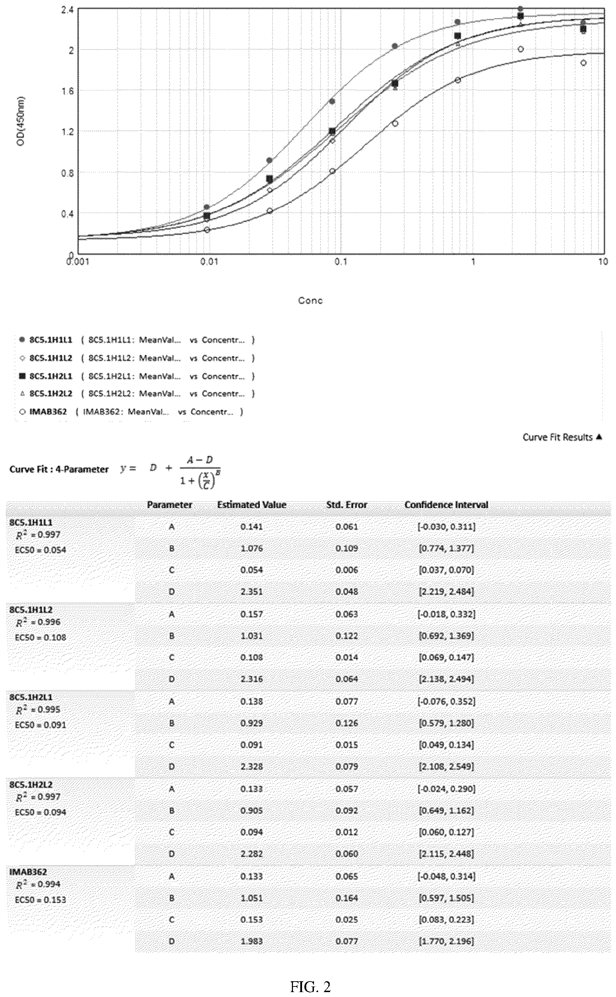

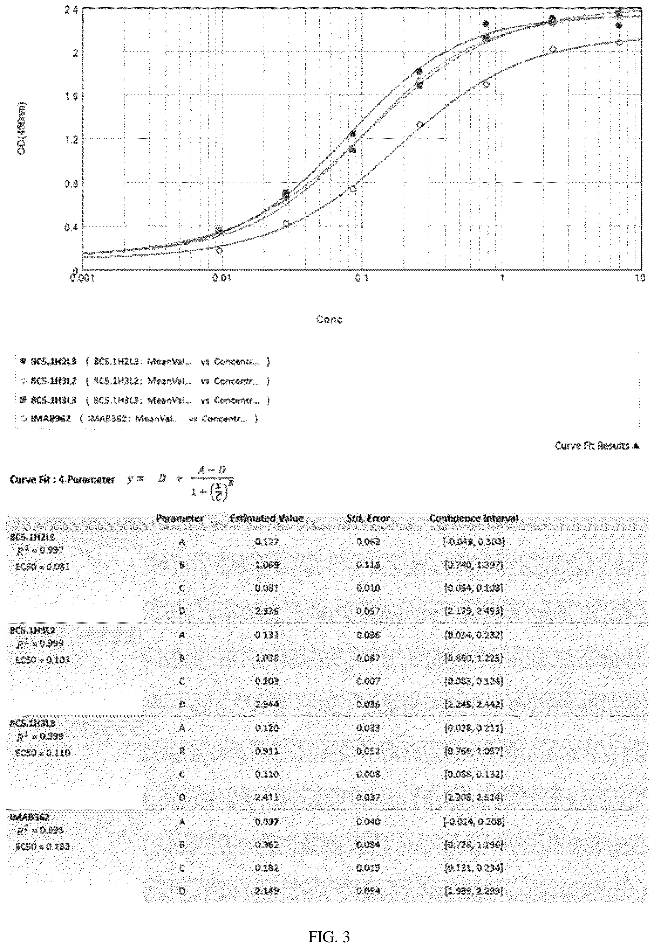

The assay results are shown in Tables 2-4 and FIGs. 1-3.

**Table 2. ELISA assay of binding of 8C5.1 to antigen on the surface of CHO-K1-CLDN18.2 cell**

| Antibody dilution | CHO-K1-CLDN18.2 | | |
|---|---|---|---|
| (µg/mL) | (20,000 cells/well) | | |
| | 8C5.1 | IMAB362 | |
| 3 | 1.613 | 1.377 | 1.411 |
| 1.000 | 1.792 | 1.485 | 1.398 |
| 0.333 | 1.634 | 1.438 | 1.413 |
| 0.111 | 1.415 | 1.224 | 1.103 |
| 0.037 | 1.071 | 0.875 | 0.799 |
| 0.012 | 0.697 | 0.456 | 0.459 |
| 0.004 | 0.344 | 0.214 | 0.213 |
| 0 | 0.086 | 0.069 | 0.072 |
| Secondary antibody | Goat anti-mouse Fc, HRP (1:5000) | Goat anti-human IgG (H+L), HRP (1:5000) | |
| EC₅₀(nM) | 0.153 | 0.201 | |

**Table 3. ELISA assay of binding of 8C5.1H1L1, 8C5.1H1L2, 8C5.1H2L1, and 8C5.1H2L2 to antigen on the surface of DN18.2 cell**

| Antibody dilution (µg/mL) | CHO-K1-CLDN18.2 (20,000 cells/well) | | | | | | | | | |
|---|---|---|---|---|---|---|---|---|---|---|
| | 8C5.1H1L1 | | 8C5.1H1L2 | | 8C5.1H2L1 | | 8C5.1H2L2 | | IMAB362 | |
| 1 | 2.250 | 2.247 | 2.224 | 2.174 | 2.235 | 2.148 | 2.203 | 2.138 | 1.898 | 1.823 |
| 0.333 | 2.431 | 2.341 | 2.332 | 2.274 | 2.329 | 2.304 | 2.242 | 2.232 | 2.021 | 1.973 |
| 0.111 | 2.276 | 2.244 | 2.131 | 2.109 | 2.164 | 2.083 | 2.075 | 2.022 | 1.644 | 1.752 |
| 0.037 | 2.064 | 1.985 | 1.695 | 1.594 | 1.678 | 1.649 | 1.580 | 1.657 | 1.257 | 1.280 |
| 0.012 | 1.496 | 1.473 | 1.114 | 1.095 | 1.192 | 1.199 | 1.166 | 1.169 | 0.755 | 0.852 |
| 0.004 | 0.985 | 0.835 | 0.629 | 0.610 | 0.746 | 0.719 | 0.708 | 0.709 | 0.410 | 0.426 |
| 0.001 | 0.452 | 0.448 | 0.330 | 0.333 | 0.371 | 0.370 | 0.376 | 0.358 | 0.237 | 0.222 |
| 0 | 0.133 | 0.129 | 0.143 | 0.135 | 0.138 | 0.122 | 0.128 | 0.130 | 0.128 | 0.115 |
| Secondary | Goat Anti Human IgG(H+L), HRP conjugated(1: 5000) | | | | | | | | | |
| antibody | | | | | | | | | | |
| EC₅₀ (nM) | 0.054 | 0.108 | 0.091 | 0.094 | 0.153 | | | | | |

**Table 4. ELISA assay of binding of 8C5.1H2L3, 8C5.1H3L2, and 8C5.1H3L3 to antigen on the surface of CHO-K1-CLDN18.2 cell**

| Antibody dilution (µg/mL) | CHO-K1-CLDN18.2 (20,000 cells/well) | | | | | | | |
|---|---|---|---|---|---|---|---|---|
| | 8C5.1H2L3 | | 8C5.1H3L2 | | 8C5.1H3L3 | | IMAB362 | |
| 1 | 2.242 | 2.237 | 2.291 | 2.296 | 2.379 | 2.308 | 2.019 | 2.142 |
| 0.333 | 2.271 | 2.335 | 2.259 | 2.247 | 2.282 | 2.257 | 1.983 | 2.057 |
| 0.111 | 2.320 | 2.177 | 2.131 | 2.150 | 2.130 | 2.119 | 1.666 | 1.724 |
| 0.037 | 1.855 | 1.768 | 1.748 | 1.731 | 1.732 | 1.642 | 1.273 | 1.392 |
| 0.012 | 1.299 | 1.177 | 1.118 | 1.069 | 1.131 | 1.073 | 0.688 | 0.797 |
| 0.004 | 0.788 | 0.624 | 0.607 | 0.624 | 0.729 | 0.621 | 0.387 | 0.471 |
| 0.001 | 0.391 | 0.306 | 0.336 | 0.342 | 0.357 | 0.341 | 0.226 | 0.121 |
| 0 | 0.110 | 0.104 | 0.113 | 0.098 | 0.115 | 0.100 | 0.110 | 0.115 |
| Secondary antibody | Goat Anti Human IgG(H+L), HRP conjugated(1: 5000) | | | | | | | |
| EC₅₀(nM) | 0.081 | | 0.103 | | 0.11 | | 0.182 | |

The results show that antibodies 8C5.1, 8C5.1H1L1, 8C5.1H1L2, 8C5.1H2L1, 8C5.1H2L2, 8C5.1H2L3, 8C5.1H3L2, 8C5.1H3L3 can all effectively bind to the antigen on the surface of the CHO-K1-CLDN18.2 cell, and the binding efficiency is in a dose-dependent manner; the binding activity of the antibodies is stronger than that of the positive control antibody IMAB362 with the same target.

### Example 2: Determination of Binding Activity of Anti-CLDN18.2 Specific Antibodies for Antigen by flow cytometry

1. Determination of binding activity of anti-CLDN18.2 specific antibody 8C5.1 for antigen by flow cytometry

CHO-K1-CLDN18.2 cells in logarithmic growth phase were collected and transferred to a 1.5 mL EP tube at 3 × 10⁵ cells/well, and then 1% PBSA was added. The mixture was centrifuged at 1000× g for 5 min, followed by removal of the supernatant. 100 µL of antibody 8C5.1 diluted with 1% PBSA (final concentrations: 30 µg/mL, 10 µg/mL, 3.33 µg/mL, 1.11 µg/mL, 0.11 µg/mL, 0.011 µg/mL, 0.0011 µg/mL, and 0.00011µg/mL) and 100 µL of antibody IMAB362 (final concentrations: 100 µg/mL, 33.3 µg/mL, 11.1 µg/mL, 3.7 µg/mL, 0.37 µg/mL, 0.037 µg/mL, 0.003 µg/mL, and 0.0003µg/mL) were each added, and the mixture was well mixed gently and incubated on ice for 1 h. 1000 µL of 1% PBSA was added, and the mixture was centrifuged at 1000× g for 5 min, followed by removal of the supernatant and washing twice with 1% PBSA. For 8C5.1, a 300-fold diluted PE Goat anti-mouse IgG (minimal x-reactivity) Antibody (Biolegend, Cat. No.: 405308) was added; for antibody IMAB362, a 300-fold diluted FITC-labeled Goat anti-human IgG secondary antibody (Jackson, Cat. No.: 109-095-098) was added. The mixture was resuspended and well mixed, and incubated on ice for 40 min in the dark. 1000 µL of 1% PBSA was added, and the mixture was centrifuged at 1000× g for 5 min, followed by removal of the supernatant and washing twice with 1% PBSA. 200 µL of 1% PBSA was added to resuspend the cell pellets, and the mixture was transferred to a flow cytometry tube for FACSCalibur assay.

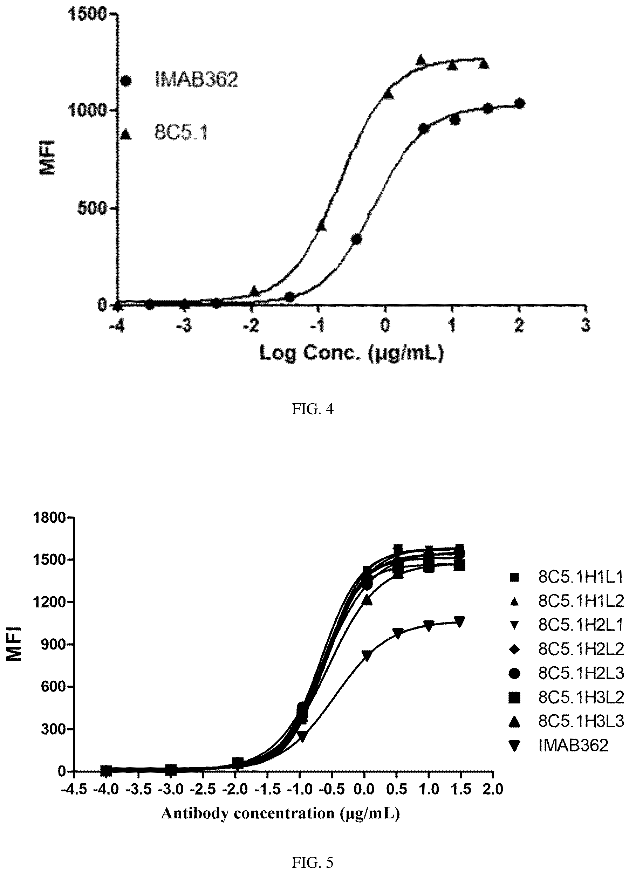

The experimental results are shown in Table 5 and FIG 4.

**Table 5. FACS assay of binding activity of 8C5.1 and IMAB362 for CLDN18.2 on the surface of CHO-K1-CLDN18.2 cell**

| Antibody | 8C5.1 | IMAB362 |
|---|---|---|
| EC_{50 (}µg/mL) | 0.2136 | 0.6873 |

### 2. Determination of binding activity of anti-CLDN18.2 specific antibodies 8C5.1H1L1, 8C5.1H1L2, 8C5.1H2L1, 8C5.1H2L2, 8C5.1H2L3, 8C5.1H3L2, and 8C5.1H3L3 for antigen by flow cytometry

CHO-K1-CLDN18.2 cells in logarithmic growth phase were collected, added to a V-bottomed 96-well plate at 3 × 10⁵ cells/well, and then centrifuged at 700× g for 5 min, followed by removal of the supernatant; diluted antibodies at corresponding concentrations were each added according to the experimental design (30 µg/mL, 10 µg/mL, 3.33 µg/mL, 1.11 µg/mL, 0.11 µg/mL, 0.011 µg/mL, 0.001 µg/mL, and 0.0001 µg/mL) at 100 µL per tube, and the mixture was incubated on ice for 1 h; 200 µL of 1% PBSA was added, and the mixture was centrifuged at 700× g for 5 min, followed by removal of the supernatant and washing three times; 100 µL of a 400-fold diluted FITC-labeled goat anti-human IgG secondary antibody (Jackson, Cat. No.: 109-095-098) was added, and the mixture was well mixed and incubated on ice for 40 min in the dark; 200 µL of 1% PBSA was added, and the mixture was centrifuged at 700× g 5 min, followed by removal of the supernatant and washing three times; 200 µL of 1% PBSA was added to resuspend the cells, and the suspension was transferred to a flow cytometry tube for testing.

The experimental results are shown in Table 6 and FIG. 5.

**Table 6. FACS assay of binding activity of 8C5.1H1L1, 8C5.1H1L2, 8C5.1H2L1, 8C5.1H2L2, 8C5.1H2L3, 8C5.1H3L2, 8C5.1H3L3, and IMAB362 for CLDN18.2 on the surface of CHO-K1-CLDN18.2 cell**

| Antibody | EC₅₀ (µg/mL) |
|---|---|
| 8C5.1H1L1 | 0.2164 |
| 8C5.1H1L2 | 0.2417 |
| 8C5.1H2L1 | 0.2668 |
| 8C5.1H2L2 | 0.2345 |
| 8C5.1H2L3 | 0.2432 |
| 8C5.1H3L2 | 0.2135 |
| 8C5.1H3L3 | 0.2808 |
| IMAB362 | 0.3581 |

The results show that, under the same experimental conditions:
EC₅₀ values for binding of 8C5.1 and IMAB362 to CHO-K1-CLDN18.2 cells were 0.2136 µg/mL and 0.6873 µg/mL, respectively;
EC₅₀ values for binding of 8C5.1H1L1, 8C5.1H1L2, 8C5.1H2L1, 8C5.1H2L2, 8C5.1H2L3, 8C5.1H3L2, 8C5.1H3L3, and IMAB362 to CHO-K1-CLDN18.2 cells were 0.2164 µg/mL, 0.2417 µg/mL, 0.2668 µg/mL, 0.2345 µg/mL, 0.2432 µg/mL, 0.2135 µg/mL, 0.2808 µg/mL, and 0.3581 µg/mL, respectively.

The above experimental results show that 8C5.1, 8C5.1H1L1, 8C5.1H1L2, 8C5.1H2L1, 8C5.1H2L2, 8C5.1H2L3, 8C5.1H3L2, 8C5.1H3L3, and IMAB362 have the activity of effectively binding to CLDN18.2 on the cell membrane surface of CHO-K1-CLDN18.2, and 8C5.1, 8C5.1H1L1, 8C5.1H1L2, 8C5.1H2L1, 8C5.1H2L2, 8C5.1H2L3, 8C5.1H3L2, and 8C5.1H3L3 have a stronger activity in binding to CLDN18.2 on the cell membrane surface of CHO-K1-CLDN18.2 than the control antibody IMAB362.

### Example 3: Assay on ADCC Activity of Anti-CLDN18.2 Antibodies

CHO-K1-CLDN18.2 cells were collected conventionally, centrifuged at 170× g for 5 min, resuspended in a medium (RPMI-1640 + 1% FBS), and then washed twice; the cells were resuspended in a medium (RPMI-1640 + 1% FBS) and counted, the cell density was adjusted, and the target cell suspension was added to a 96-well plate at 100 µL/well (approximately 3 × 10⁴ target cells per well); antibodies 8C5.1H1L1, 8C5.1H1L2, 8C5.1H2L1, 8C5.1H2L2, 8C5.1H2L3, 8C5.1H3L2, 8C5.1H3L3, and IMAB362 were diluted with a medium (RPMI-1640 + 1% FBS) (working concentrations were all 30 µg/mL, 10 µg/mL, 3.33 µg/mL, 1.11 µg/mL, 0.11 µg/mL, 0.011 µg/mL, 0.001 µg/mL, and 0.0001 µg/mL), and the diluted antibodies were each added to the corresponding wells at 50 µL/well, followed by incubation in an incubator at 37 °C for 1 h; meanwhile, a negative control medium and an isotype control hIgG1 (produced by Akeso Biopharma Inc., Batch No.: 20190410) were set.

Effector cells PBMC (from a healthy donor) were collected conventionally and centrifuged at 170× g for 5 min, followed by removal of the supernatant and washing twice; the cells were resuspended in a medium (RPMI-1640 + 1% FBS) and counted, and the cell density was adjusted; 50 µL of the effector cell suspension was added to the pre-incubated target cells (containing about 6 × 10⁵ effector cells per well), and the mixture was well mixed, incubated in an incubator at 5% CO₂ and 37 °C for 4 h, and then centrifuged at 250× g for 5 min; 100 µL of culture supernatant was carefully pipetted into a new 96-well flat-bottom plate, 100 µL of a newly prepared reaction solution was added to each well, and the mixture was incubated for 30 min at room temperature in the dark; OD values were measured at 490 nm and 650 nm, and OD value for each group = OD_{490 nm} - OD_{650 nm}.

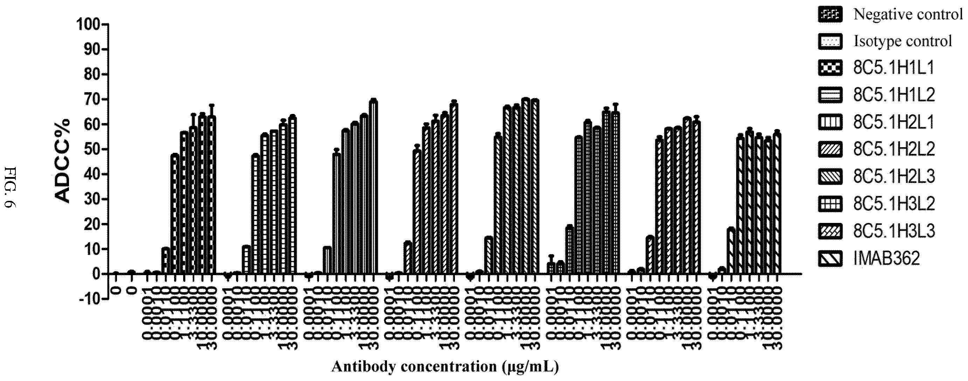

The results are shown in FIG. 6.

The results show that compared with the isotype control, the anti-CLDN18.2 antibodies 8C5.1H1L1, 8C5.1H1L2, 8C5.1H2L1, 8C5.1H2L2, 8C5.1H2L3, 8C5.1H3L2 and 8C5.1H3L3 and the positive control antibody IMAB362 have ADCC activity and are capable of specifically killing CLDN18.2-positive cells, and they have comparable killing activity.

### Example 4: Assay on CDC Activity of Anti-CLDN18.2 Antibodies

CHO-K1-CLDN18.2 cells were digested and collected conventionally, centrifuged at 170× g for 5 min, resuspended in a medium (RPMI-1640 + 1% FBS), and then washed twice; the cells were resuspended in a medium (RPMI-1640 + 1% FBS) and counted, the cell density was adjusted, and the target cell suspension was added to a 96-well plate at 100 µL/well (approximately 3 × 10⁴ cells per well); antibodies were diluted with a medium (RPMI-1640 + 1% FBS) (working concentrations were all 30 µg/mL, 3.33 µg/mL, 0.11 µg/mL, 0.001 µg/mL, and 0.0001 µg/mL), and the diluted antibodies were each added to the corresponding wells at 50 µL/well, and the mixture was preincubated at room temperature for 10 min; 50 µL of complement serum (final concentration: 2%) was added to the preincubated target cells, and the mixture was well mixed, incubated in an incubator at 5% CO₂ and 37 °C for 4 h, and then centrifuged at 250× g for 5 min; 100 µL of culture supernatant was carefully pipetted into a new 96-well flat-bottom plate, 100 µL of a newly prepared reaction solution was added to each well, and the mixture was incubated for 30 min at room temperature in the dark; OD values were measured at 490 nm and 650 nm, and OD value for each group = OD_{490 nm} - OD_{650 nm}.

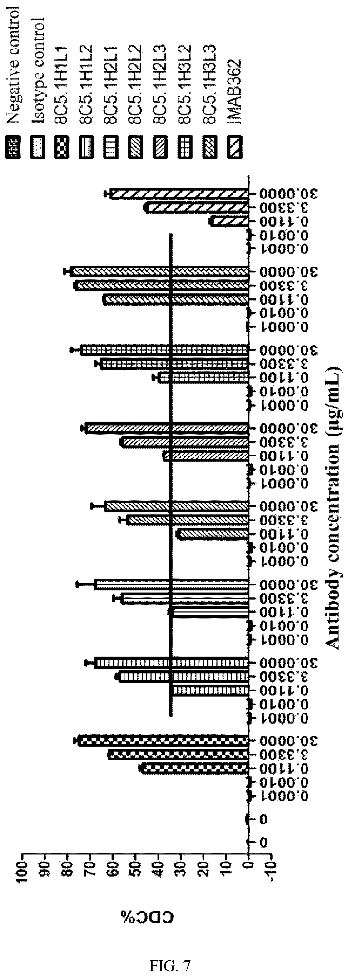

The results are shown in FIG. 7.

The results showed that compared with the isotype control (produced by Akeso Biopharma Inc., Batch No.: 20190410), the anti-CLDN18.2 antibodies 8C5.1H1L1, 8C5.1H1L2, 8C5.1H2L1, 8C5.1H2L2, 8C5.1H2L3, 8C5.1H3L2, and 8C5.1H3L3 all have CDC activity and are capable of killing tumor cells under mediation of the complement, and their activity is better than that of the positive control antibody IMAB362.

### Example 5: Anti-CLDN18.2 Antibodies Promoting Phaeocytosis of Tumor Cells by Macrophages

In this example, the antibody-dependent cellular phagocytosis (ADCP) activity mediated by the antibody 8C5.1H3L3 was detected using MBMMs (mouse bone marrow-derived macrophages) as effector cells and CHO-K1-CLDN18.2 as target cells.

The specific method of this example is as follows:
CHO-K1-CLDN18.2 was collected conventionally, centrifuged at 170× g for 5 min, resuspended, counted, analyzed for viability, and washed once with PBS. CFSE was diluted to 2.5 µM with PBS, and the cells were resuspended in a proper amount of the diluted CFSE (staining density: 10 million cells/mL) and incubated in an incubator for 20 min.

6 mL of a DMEM complete medium (containing 10% of FBS) was added to stop the staining. The cells were centrifuged at 170× g for 5 min, followed by removal of the supernatant. 1 mL of a DMEM complete medium was added and the cells were incubated in an incubator for 10 min. The antibody was diluted with a DMEM complete medium (10 µg/mL, 1 µg/mL and 0.1 µg/mL), and an isotype control antibody and a reference antibody were designed.

Macrophages were collected and centrifuged at 170× g for 5 min, followed by removal of the supernatant and cell counting; the cells were transferred to a 1.5 mL EP tube, centrifuged at 1200× g for 5 min, followed by removal of the supernatant; the tumor cell and antibody suspension incubated for 30 min were added to a 1.5 mL EP tube containing macrophages, followed by re-suspending and well mixing, and the mixture was incubated in an incubator at 37 °C for 2 h; 800 µL of normal-temperature 1% PBSA was added to each tube, and the mixture was centrifuged at 1200× g for 5 min, followed by removal of the supernatant and washing once with 800 µL of PBSA; a 600-fold diluted APC anti-mouse/human CD11b antibody (Biolegend, Cat. No.: 101212) was added to the corresponding samples at 100 µL/sample, and the mixture was well mixed and incubated on ice for 40 min. 800 µL of 1% PBSA was added to each tube, and the mixture was centrifuged at 1200× g for 5 min, followed by removal of the supernatant; each tube was washed once with 200 µL of PBSA, and 200 µL of 1% PBSA was added to each tube for resuspending. The mixture was then transferred to a flow cytometry tube for testing.

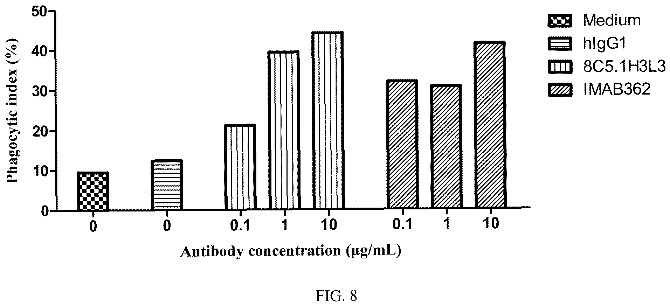

The results are shown in FIG. 8.

The results show that the phagocytic indexes of 8C5.1H3L3 and IMAB362 (reference antibody) (produced by Akeso Biopharma Inc., Batch No.: 20190704) were significantly higher than that of the blank control and the isotype control, indicating that 8C5.1H3L3 and the reference antibody IMAB362 both have ADCP activity and that 8C5.1H3L3 has better ADCP activity than IMAB362.

Although specific embodiments of the present invention have been described in detail, those skilled in the art will appreciate that various modifications and substitutions can be made to those details according to all the teachings that have been disclosed, and these changes shall all fall within the protection scope of the present invention. The full scope of the present invention is given by the appended claims and any equivalent thereof.

## Claims

1. An anti-CLDN18.2 antibody or an antigen-binding fragment thereof, wherein the anti-CLDN18.2 antibody comprises a heavy chain variable region comprising HCDR1 to HCDR3 and a light chain variable region comprising LCDR1 to LCDR3, wherein:
an amino acid sequence of HCDR1 is set forth in SEQ ID NO: 5, an amino acid sequence of HCDR2 is set forth in SEQ ID NO: 6, and an amino acid sequence of HCDR3 is set forth in SEQ ID NO: 7, and
an amino acid sequence of LCDR1 is set forth in SEQ ID NO: 8, an amino acid sequence of LCDR2 is set forth in SEQ ID NO: 9, and an amino acid sequence of LCDR3 is set forth in SEQ ID NO: 10.

2. The anti-CLDN18.2 antibody or the antigen-binding fragment thereof according to claim 1, wherein,
an amino acid sequence of the heavy chain variable region of the anti-CLDN18.2 antibody is selected from SEQ ID NO: 2, SEQ ID NO: 11, SEQ ID NO: 13, and SEQ ID NO: 15; and
an amino acid sequence of the light chain variable region of the anti-CLDN18.2 antibody is selected from SEQ ID NO: 4, SEQ ID NO: 17, SEQ ID NO: 19, and SEQ ID NO: 21.

3. The anti-CLDN18.2 antibody or the antigen-binding fragment thereof according to any one of claims 1-2, wherein,
the amino acid sequence of the heavy chain variable region is set forth in SEQ ID NO: 2, and the amino acid sequence of the light chain variable region is set forth in SEQ ID NO: 4;
the amino acid sequence of the heavy chain variable region is set forth in SEQ ID NO: 11, and the amino acid sequence of the light chain variable region is set forth in SEQ ID NO: 17;
the amino acid sequence of the heavy chain variable region is set forth in SEQ ID NO: 11, and the amino acid sequence of the light chain variable region is set forth in SEQ ID NO: 19;
the amino acid sequence of the heavy chain variable region is set forth in SEQ ID NO: 11, and the amino acid sequence of the light chain variable region is set forth in SEQ ID NO: 21;
the amino acid sequence of the heavy chain variable region is set forth in SEQ ID NO: 13, and the amino acid sequence of the light chain variable region is set forth in SEQ ID NO: 17;
the amino acid sequence of the heavy chain variable region is set forth in SEQ ID NO: 13, and the amino acid sequence of the light chain variable region is set forth in SEQ ID NO: 19;
the amino acid sequence of the heavy chain variable region is set forth in SEQ ID NO: 13, and the amino acid sequence of the light chain variable region is set forth in SEQ ID NO: 21;
the amino acid sequence of the heavy chain variable region is set forth in SEQ ID NO: 15, and the amino acid sequence of the light chain variable region is set forth in SEQ ID NO: 17;
the amino acid sequence of the heavy chain variable region is set forth in SEQ ID NO: 15, and the amino acid sequence of the light chain variable region is set forth in SEQ ID NO: 19;
or
the amino acid sequence of the heavy chain variable region is set forth in SEQ ID NO: 15, and the amino acid sequence of the light chain variable region is set forth in SEQ ID NO: 21.

4. The anti-CLDN18.2 antibody or the antigen-binding fragment thereof according to any one of claims 1-3, wherein for the antibody, a heavy chain constant region is an Ig gamma-1 chain C region or an Ig gamma-4 chain C region, and a light chain constant region is an Ig kappa chain C region.

5. The anti-CLDN18.2 antibody or the antigen-binding fragment thereof according to any one of claims 1-4, wherein the anti-CLDN18.2 antibody or the antigen-binding fragment thereof is selected from Fab, Fab', F(ab')₂, Fd, Fv, dAb, a complementarity determining region fragment, a single chain fragment variable, a humanized antibody, or a chimeric antibody.

6. The anti-CLDN18.2 antibody or the antigen-binding fragment thereof according to any one of claims 1-5, wherein,
the antibody comprises a non-CDR region derived from a non-murine species, such as from a human antibody.

7. The anti-CLDN18.2 antibody or the antigen-binding fragment thereof according to any one of claims 1-6, wherein:
an EC₅₀ for binding of the anti-CLDN18.2 antibody to a cell expressing CLDN18.2 is less than or equal to 0.5 µg/mL, less than or equal to 0.4 µg/mL, or less than or equal to 0.3 µg/mL;
preferably, the EC₅₀ is measured by FACS.

8. The antibody or the antigen-binding fragment thereof according to any one of claims 1-7, wherein the antibody is a monoclonal antibody produced by a hybridoma cell line LT020 deposited at China Center for Type Culture Collection (CCTCC) with a CCTCC designation of CCTCC NO. C2022124.

9. An isolated nucleic acid molecule encoding the anti-CLDN18.2 antibody or the antigen-binding fragment thereof according to any one of claims 1-8.

10. A recombinant vector, comprising the isolated nucleic acid molecule according to claim 9.

11. A host cell, comprising the isolated nucleic acid molecule according to claim 9 or the recombinant vector according to claim 10.

12. A hybridoma cell line LT020 deposited at China Center for Type Culture Collection (CCTCC) with a CCTCC designation of CCTCC NO. C2022124.

13. An antibody-drug conjugate, comprising an antibody or an antigen-binding fragment thereof and a small molecule drug, wherein the antibody or the antigen-binding fragment thereof is the anti-CLDN18.2 antibody or the antigen-binding fragment thereof according to any one of claims 1-8; preferably, the small molecule drug is a small molecule cytotoxic drug; more preferably, the small molecule drug is an anti-tumor chemotherapeutic drug.

14. The antibody-drug conjugate according to claim 13, wherein the antibody or the antigen-binding fragment thereof is linked to the small molecule drug via a linker; for example, the linker is a hydrazone bond, a disulfide bond, or a peptide bond;
preferably, a molar ratio of the antibody or the antigen-binding fragment thereof to the small molecule drug is 1:(2-4).

15. A pharmaceutical composition, comprising an effective amount of the anti-CLDN18.2 antibody or the antigen-binding fragment thereof according to any one of claims 1-8 or the antibody-drug conjugate according to any one of claims 13-14, wherein optionally, the pharmaceutical composition further comprises one or more pharmaceutically acceptable auxiliary materials.

16. Use of the anti-CLDN18.2 antibody or the antigen-binding fragment thereof according to any one of claims 1-8 in preparing a medicament for treating or preventing a tumor, wherein,
preferably, the tumor is a CLDN18.2-positive tumor;
preferably, the tumor is one or more selected from biliary tract cancer, bronchogenic carcinoma, lymphoma, ovarian cancer, esophageal cancer, melanoma, a hematological malignancy, glioblastoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer.

17. The anti-CLDN18.2 antibody or the antigen-binding fragment thereof according to any one of claims 1-8 for use in treating or preventing a tumor, wherein,
preferably, the tumor is a CLDN18.2-positive tumor;
preferably, the tumor is one or more selected from biliary tract cancer, bronchogenic carcinoma, lymphoma, ovarian cancer, esophageal cancer, melanoma, a hematological malignancy, glioblastoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer.

18. A method for treating or preventing a tumor, comprising the step of administering to a subject in need thereof an effective amount of the anti-CLDN18.2 antibody or the antigen-binding fragment thereof according to any one of claims 1-8, wherein,
preferably, the tumor is a CLDN18.2-positive tumor;
preferably, the tumor is one or more selected from biliary tract cancer, bronchogenic carcinoma, lymphoma, ovarian cancer, esophageal cancer, melanoma, a hematological malignancy, glioblastoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer.

19. The method for treating or preventing a tumor according to claim 18, wherein drug administration is performed before or after surgery and/or before or after radiotherapy.

20. The method for treating or preventing a tumor according to any one of claims 18-19, wherein,
a dose for each administration of the anti-CLDN18.2 antibody or the antigen-binding fragment thereof is 0.1-100 mg per kg body weight, preferably 5-50 mg or 5-15 mg per kg body weight;
preferably, drug administration is performed once every 3 days, every 4 days, every 5 days, every 6 days, every 10 days, every 1 week, every 2 weeks, or every 3 weeks;
preferably, a route of administration is intravenous drip infusion or intravenous injection.
